(19) Europäisches Patentamt
European Patent Office
Office européen des brevets

(11) EP 3 075 853 B1

(12) **EUROPEAN PATENT SPECIFICATION**

(45) Date of publication and mention of the grant of the patent:
**06.12.2017 Bulletin 2017/49**

(21) Application number: **16163068.6**

(22) Date of filing: **31.03.2016**

(51) Int Cl.:
***C12N 15/09*** *(2006.01)* ***C12Q 1/04*** *(2006.01)*
***C12Q 1/68*** *(2006.01)*

(54) **METHOD OF OBTAINING INFORMATION ON BREAST CANCER AND KIT FOR DETECTING BREAST CANCER**

VERFAHREN ZUR GEWINNUNG VON INFORMATIONEN ÜBER BRUSTKREBS UND KIT ZUM NACHWEIS VON BRUSTKREBS

PROCÉDÉ D’OBTENTION D’INFORMATIONS SUR LE CANCER DU SEIN ET KIT DE DÉTECTION DU CANCER DU SEIN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.03.2015 JP 2015073577**

(43) Date of publication of application:
**05.10.2016 Bulletin 2016/40**

(73) Proprietors:
- **SYSMEX CORPORATION**
  **Kobe-shi,**
  **Hyogo 651-0073 (JP)**
- **The University of Tokyo**
  **Bunkyo-ku,**
  **Tokyo 113-8654 (JP)**

(72) Inventors:
- **TAI, Kaya**
  **Kobe-shi**
  **Hyogo 651-0073 (JP)**
- **NAGAE, Genta**
  **Tokyo 113-8654 (JP)**
- **ABURATANI, Hiroyuki**
  **Tokyo 113-8654 (JP)**

(74) Representative: **Paemen, Liesbet R.J. et al**
**De Clercq & Partners**
**Edgard Gevaertdreef 10 a**
**9830 Sint-Martens-Latem (BE)**

(56) References cited:
**EP-A1- 2 341 150** **US-A1- 2011 318 738**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 075 853 B1

## Description

### TECHNICAL FIELD

The present invention relates to a method of obtaining information on breast cancer in a subject and a kit for detecting breast cancer.

### BACKGROUND

Breast cancer is a malignant tumor that occurs in the mammary duct or gland. Breast cancer is known as the leading cause of death from cancer in women. For screening for breast cancer, clinical breast examination for detection of any lump in the breast and mammography, i.e., X-ray photography of the breast are performed. When any lump is found in a subject, an imaging method (e.g., ultrasonography) and fine-needle aspiration cytology are performed. However, these examinations may result in discomfort or pain in the subject.

For screening for breast cancer, measurement of tumor markers such as carcinoembryonic antigen (CEA) and CA 15-3 is also performed. However, these tumor markers have insufficient sensitivity in detecting cancer. The tumor markers include markers used for cancers of different types from breast cancer.

New methods for diagnosing cancer based on genetic information have been studied in recent years. The methods include, for example, a method based on information on methylation of DNA. In this method, CpG sites (5'-(CG)-3') in base sequences of certain genes are used as markers. Then, information such as the presence or absence of cancer cells is obtained based on the analysis results of the methylation status of the markers. Information is used as an index for diagnosis of cancer. For example, Dulaimi E. et al.,Tumor Suppressor Gene Promoter Hypermethylation in Serum of Breast Cancer Patients, Clin Cancer Res, vol. 10, p. 6189-6193 (2004) discloses that the promoters of RASSF1A, APC, and DAP-kinase genes are highly methylated in the serum of patients with breast cancer and the methylation analyses indicate the usefulness of the markers in screening for breast cancer. US 2011/318738 describes RIIAD1 (c1orf230) as one of differentially methylated genes in adenoma. EP 2 341 150 describes methylation markers for breast cancer.

### SUMMARY OF THE INVENTION

However, the number of genes used as markers for detecting breast cancer is few. The present inventors have identified that, among the markers disclosed in Dulaimi E. et al.,Tumor Suppressor Gene Promoter Hypermethylation in Serum of Breast Cancer Patients, Clin Cancer Res, vol. 10, p. 6189-6193 (2004), the markers of RASSF1A and APC genes are highly methylated not only in breast cancer tissues but also various other cancer tissues. Thus, there is a demand for development of novel markers for detecting breast cancer using methylation analysis of genes.

There is provided a method for obtaining information for breast cancer in a subject. The method comprises steps of: analyzing methylation status of a CpG site located in a promoter region of RIIAD1 gene in a DNA sample prepared from a biological sample of said subject; and obtaining information indicating the presence of breast cancer cells in the biological sample when the result in the determining step indicates the presence of a methylated CpG site. In particular embodiments, the methylation status of SEQ ID NO: 7 is analyzed in the analyzing step. In particular embodiments, the analyzing step is conducted by at least one method selected from the group consisting of mass spectrometry and methylation-specific PCR method. In particular embodiments, the biological sample is a tissue, blood or serum. In particular embodiments, the analyzing step comprises: calculating methylation frequency of the promoter region, and the obtaining step comprises: comparing the frequency with a predetermined threshold; and obtaining information indicating the presence of breast cancer cells in the biological sample when the frequency is equal to or higher than the threshold. In particular embodiments, the method comprises preparing a DNA sample from the biological sample. In further particular embodiments, the preparing step comprises treating DNA in the biological sample with bisulfite.

There is provided the use of a reagent kit for detecting breast cancer, said kit comprising a primer that hybridizes to a promotor region of RIIAD1 gene, said promoter region comprising: a uracil base converted from unmethylated cytosine base by a bisulfite treatment; and methylated cytosine base, wherein the primer is used for analyzing methylation status of a CpG site in the promoter region of RIIAD1 gene in a DNA sample. In particular embodiments, the primer is a primer comprising a base sequence SEQ ID NO: 3 or 4.

### BRIEF DESCRIPTION OF THE DRAWINGS

Fig. 1 is a graph illustrating a methylation positive rate in a promoter region of RIIAD1 gene calculated from methylation data of cancerous tissues and non-cancerous tissues of breast cancer, and normal mammary tissues;
Fig. 2 is a graph illustrating a methylation positive rate in a promoter region of RIIAD1 gene calculated from methylation data of various clinical samples;
Fig. 3 is an image describing the results of methylation-specific PCR (MSP) amplification of DNA extracted from normal mammary tissues and cancerous tissues derived from a breast cancer patient;
Fig. 4 is an image describing the results of MSP amplification of DNA extracted from various cancer tissues and normal tissues from various organs;
Fig. 5 is a schematic view illustrating one example of a determination device for providing information on breast cancer in a subject;
Fig. 6 is a block diagram illustrating the functional configuration of the determination device of Fig. 5;
Fig. 7 is a block diagram illustrating the hardware configuration of the determination device illustrated in Fig. 5; and
Fig. 8 is a flow chart of determination for providing information on breast cancer in a subject using the determination device illustrated in Fig. 5.

DETAILED DESCRIPTION OF THE PREFERRED EMBODIMENTS

In the method for obtaining information on breast cancer envisaged herein (hereinafter also simply referred to as "method"), information is obtained from a DNA sample prepared from a biological sample collected from a subject. In particular embodiments, the method also comprises the step of preparing a DNA sample from a biological sample obtained from a subject.

In the methods envisaged herein, the biological sample is not particularly limited as long as it is a biological sample containing DNA of a subject. However, the biological sample is preferably a sample containing a genomic DNA such as a clinical specimen. Examples of the clinical specimen include body fluid, urine, cells obtained by fine-needle aspiration, and tissues obtained by operations or biopsies. Examples of the body fluid include blood, serum, plasma, lymph fluid, ascitic fluid, bone marrow fluid, and nipple discharge. The biological sample may also be a culture obtained by culturing cells or tissues collected from a subject. Further, the biological sample may be a formalin-fixed paraffin-embedded (FFPE) tissue sample collected from a subject.

The DNA sample can be prepared by extracting DNA from the biological sample. A method for extracting DNA from a biological sample is well-known in the art. DNA can be extracted by, for example, mixing the biological sample with a treatment solution containing a surfactant for solubilization of cells or tissues (such as sodium cholate and sodium dodecyl sulfate) and subjecting the resulting mixture to physical procedure (such as stirring, homogenization, and ultrasonication) to release DNA contained in the biological sample into the mixture. In this case, a supernatant containing DNA released by centrifuging the mixture to precipitate cell debris is preferably used in a later-described analyzing step. The obtained supernatant may be purified by any well-known method in the art. DNA can also be extracted from the biological sample and purified by using a commercially-available kit.

Preferably, the above-described preparing step further includes a step of fragmenting the extracted DNA. By fragmenting the DNA to have appropriate length, methylated DNA immunoprecipitation (MeDIP) and non-methylated cytosine converting treatment as described below can be effectively performed. Fragmentation of DNA may be performed by ultrasonication, alkaline treatment, restriction enzyme treatment, or the like. When DNA is fragmented by alkaline treatment, for example, a sodium hydroxide solution is added to a DNA solution to obtain a final concentration of 0.1 to 1.0 N and the mixture is incubated at 10 to 40°C for 5 to 15 minutes to fragment the DNA. When DNA is fragmented by the restriction enzyme treatment, the restriction enzyme is appropriately selected based on the base sequence of DNA, which may be MseI or BamHI, for example.

The methods of the present invention involve determining the presence or absence of methylation of a CpG site located in a promotor region of RIIAD1 gene included in the DNA obtained from the biological sample. The term "CpG site" used herein means a site of a sequence in which cytosine (C) and guanine (G) are adjacent in this order from 5' to 3' in the base sequence. The letter "p" in "CpG" represents a phosphodiester bond between cytosine and guanine.

The base sequence of the promoter region of RIIAD1 gene itself is well-known in the art. The base sequence can be obtained from a well-known database provided by, for example, the National Center for Biotechnology Information (NCBI) (http://www.ncbi.nlm.nih.gov/) of the National Library of Medicine. The ID number of RIIAD1 gene is shown in Table 1. The base sequence of the promotor region of the RIIAD1 gene is represented by SEQ ID NO: 1 (chr1: 151692000-151696132).

[Table 1]

| Gene symbol | Unigene ID | Entrez Gene ID | SEQ ID NO: |
|---|---|---|---|
| *RIIAD1* | HS.297967 | 284485 | 1 |

In particular embodiments of the methods envisaged herein, the presence or absence of methylation of a CpG site can be analyzed based on the analysis result on methylation status in the promotor region. The methylation analysis can be performed by analyzing the presence or absence of methylation of cytosine located in at least one CpG site among CpG sites located in a promoter region of RIIAD1 gene. In this case, only one CpG site may be analyzed, but a plurality of CpG sites is preferably analyzed for the presence or absence of methylation.

In a particular embodiment, methylation analysis may be performed by analyzing methylation frequency in a promoter region of RIIAD1 gene. The term "methylation frequency" means a ratio of the number of methylated CpG sites relative to the number of CpG sites located in the promoter region. In this embodiment, a target for analysis may be the entire promoter region or a part of the promoter region including at least one CpG site. The target for analysis may contain only one CpG site, but the target for analysis preferably contains a plurality of CpG sites. The position and number of CpG sites located in the promoter region of RIIAD1 gene are already known, and thus, in the embodiment, the number of methylated CpG sites itself in the promoter region can be used as the methylation frequency.

The methylation frequency may be a "methylation score" obtained by analyzing methylation status of a CpG site in DNA with mass spectrometry such as MassARRAY (registered trademark) as described below. MassARRAY (registered trademark) allows calculation of a methylation score based on a ratio between the area of a peak derived from methylated DNA fragment and the area of a peak derived from non-methylated DNA fragment obtained through measurement of DNA fragments.

In particular embodiments, the methylation frequency in a promotor region of RIIAD1 gene may be calculated by a hand method or a machine such as a computer.

In the methods envisaged herein, the target for analysis is not particularly limited, and may be any CpG sites (or certain regions including the CpG sites) in the promoter region of RIIAD1 gene. The target for analysis may be appropriately selected by a person skilled in the art. The positions and number of CpG sites located in the promoter region of the gene are already known. Thus, the target CpG sites or regions may be selected by routine experiments according to the well-known analysis method described below.

Various methods for analyzing methylation status are well-known in the art. The analysis method to be used in the methods envisaged herein is not particularly limited. However, the analysis method preferably includes a step of differentiating methylated DNA from non-methylated DNA, a step of amplifying DNA, and a step of detecting methylated DNA and/or non-methylated DNA.

The step of differentiating methylated DNA from non-methylated DNA may include a step of performing methylation sensitive restriction enzyme treatment, a MeDIP method, non-methylated cytosine converting treatment, or the like. The step of amplifying DNA may include a step of performing PCR, quantitative PCR, IVT (in vitro transcription) amplification, SPIA (trademark) amplification methods, or the like. The step of detecting methylated DNA and/or non-methylated DNA may include a step of performing electrophoresis, sequence analysis, microarray analysis, mass spectrometry, Southern hybridization, or the like.

The MeDIP method is used to enrich methylated DNA in a biological sample by immunoprecipitation using an anti-methylated cytosine antibody or an anti-methylated cytidine antibody, or an antibody which specifically recognizes a methylated DNA-binding protein. In the methods envisaged herein, methylation analysis may be performed by enriching methylated DNA in DNA obtained in the extracting step by the MeDIP method and analyzing methylation status of the obtained methylated DNA. The methylated DNA enriched by the MeDIP method may be amplified by, for example, IVT amplification, and the methylation status of the obtained amplified product may be analyzed by using a microarray. This analysis method is referred to as "MeDIP on chip."

The non-methylated cytosine converting treatment is used to react DNA extracted from a biological sample with a non-methylated cytosine conversion agent so as to convert non-methylated cytosine in the DNA to another base (uracil, thymine, adenine or guanine). The non-methylated cytosine conversion agent is a substance that can react with DNA and convert non-methylated cytosine in the DNA to another base (uracil, thymine, adenine or guanine). The non-methylated cytosine conversion agent to be preferably used is bisulfite such as sodium, potassium, calcium or magnesium bisulfite.

In the treatment of DNA using bisulfite, non-methylated cytosine in the DNA is converted to uracil due to deamination reaction, while methylated cytosine does not undergo such a base conversion. Thus, the difference in methylation status of a CpG site in DNA is converted to the difference in a base sequence (C and U) by the non-methylated cytosine converting treatment using bisulfite. The non-methylated cytosine converting treatment using bisulfite is referred to as "bisulfite treatment."

When the bisulfite treatment is performed, the additive amount (concentration) of bisulfite is not specifically limited as long as it can sufficiently convert non-methylated cytosine in DNA. For example, the final concentration in a solution containing DNA is higher than or equal to 1 M, preferably 1 M to 15 M, and more preferably 3 M to 10 M. The incubation condition (temperature and time) after addition of bisulfite may be appropriately selected depending on the additive amount of bisulfite. For example, when bisulfite is added at a final concentration of 6 M, the incubation is carried out at 50 to 80°C for 10 to 90 minutes.

Methylation status of CpG sites in DNA can be analyzed by analyzing the sequence of DNA after bisulfite treatment and detecting the difference in base sequence from the original sequence. This method is referred to as "bisulfite sequencing."

The methylation status of CpG sites can be analyzed by mass spectrometry. Specifically, DNA after bisulfite treatment as a template is amplified by PCR using a primer set specific for a base sequence which is a target for analysis, and the obtained PCR product is subjected to IVT amplification to convert methylated cytosine and uracil respectively to guanine (G) and adenine (A). The obtained IVT amplification product is cleaved with RNase A, and the difference in mass (16 Da) due to difference between G and A in the obtained nucleic acid fragments is detected using a MALDI-TOF (matrix assisted laser desorption/ionization time-of-flight) mass spectrometer to analyze methylation status of the DNA. This method is referred to as "MassARRAY (registered trademark) analysis."

It is known that the site of IVT product cleaved with RNase A is between an arbitrary base sequence and the adjacent uracil (U) or thymine (T). Thus, the base sequence and mass of the IVT product cleaved with RNase A can be predicted based on the base sequence of the template DNA. Accordingly, it is possible to identify a portion of the base sequence of the template DNA from which each peak obtained in MassARRAY (registered trademark) is originated. For example, when one CpG site is methylated in a DNA fragment, a peak obtained in MassARRAY (registered trademark) shifts to the side with an increased mass for 16 Da. In analysis of a DNA fragment containing a plurality of CpG sites, for example, a shift of 32 Da is shown when two CpG sites in the DNA fragment are methylated, and a shift of 48 Da is shown when three methylated CpG sites in the DNA fragment are methylated.

In mass spectrometry such as MassARRAY (registered trademark), the methylation score of the analyzed DNA fragment can be calculated. For example, when the ratio between the area of the peak of the non-methylated DNA fragment and the area of the peak of the methylated DNA fragment in a chart obtained from the analysis of a DNA fragment having a certain sequence is 1 : 3, the methylation score of the DNA fragment is 0.75 (= 3/(1 + 3)). The methylation score is theoretically 1 when all CpG sites in the DNA fragment are methylated, and 0 when not all CpG sites in the DNA fragment are methylated.

The methylation status of CpG sites can be analyzed by a methylation-specific PCR (MSP) method. The MSP method is a method of analyzing the presence or absence of methylation of CpG sites by amplifying DNA after bisulfite treatment by PCR using a primer set described below and determining the presence or absence of a PCR product. The MSP method utilizes a primer set that can amplify a base sequence where a CpG site to be analyzed is methylated (i.e. cytosine is not converted to uracil), but cannot amplify a base sequence where a CpG site is not methylated (i.e. cytosine is converted to uracil). According to the MSP method using such a primer set, the presence of a PCR product indicates the methylation of the CpG site to be analyzed. The MSP method may also utilize a primer set that cannot amplify a base sequence where cytosine in a CpG site to be analyzed is not converted to uracil, but can amplify a base sequence where cytosine in a CpG site is converted to uracil. In this case, the absence of a PCR product indicates the methylation of the CpG site to be analyzed.

In particular embodiments, the presence or absence of a PCR product can be verified by gel electrophoresis, for example. The reaction solution after amplification is subjected to gel electrophoresis, and then the presence or absence of a PCR product band in the gel may be visually confirmed. Alternatively, an image of the gel after electrophoresis is obtained, and then the resulting image may be analyzed by image analysis. The image analysis can be performed, for example, by obtaining the gray value (e.g., band intensity) of pixels in the region where the PCR product is assumed to be present in the gel image and comparing the value to a predetermined threshold. Specifically, when the gray value is higher than or equal to the predetermined threshold, the result indicates the presence of a PCR product. When the gray value is lower than the predetermined threshold, the result indicates the absence of a PCR product. The predetermined threshold for the shading of the pixels is not particularly limited, and may be for example, a value indicating the gray value in pixels in the background or a value double or triple the above value.

Each primer in the primer set used for the MSP method may be appropriately designed by a person skilled in the art based on the base sequence including a CpG site to be analyzed. Each primer is preferably designed so as to contain cytosine of the CpG site to be analyzed at the 3' end of the primer or in the vicinity thereof.

The methylation status of CpG sites may alternatively be analyzed with a microarray. In this case, the microarray for analysis may be prepared by immobilizing a nucleic probe complementary to the base sequence of a promoter region of RIIAD1 gene on a substrate. The microarray can be prepared according to a well-known method in the art.

In the analysis using a microarray, DNA extracted from a biological sample is preferably labeled with a labeling substance well-known in the art. Thus, the determination method of the methods envisaged herein preferably further includes a step of labeling the extracted DNA. The labeling step is advantageously carried out after the DNA amplifying step because all DNA in the biological sample can be labeled. Examples of the labeling substance include fluorescent substances, haptens such as biotin, and radioactive substances. Examples of the fluorescent substances include $Cy^3$, $Cy^5$, FITC, and Alexa Fluor (trademark). Labeling of DNA facilitates measurement of a signal from a probe on the microarray. The method for labeling DNA with the labeling substance is well-known in the art.

The above signal may be any suitable signal depending on the type of microarrays. For example, the signal may be an electric signal generated when a DNA fragment hybridizes to each probe on the microarray or a fluorescence or luminescence signal generated from a labeling substance when DNA to be analyzed is labeled as described above. The signal can be detected using a scanner included in a normal microarray analyzer. Examples of the scanner include GeneChip (registered trademark) Scanner3000 7G (Affymetrix, Inc.), and Illumina (registered trademark) BeadArray Reader (Illumina, Inc.).

In particular embodiments, when the analysis result in the methylation analysis indicates the presence of methylated CpG sites, the presence of methylated CpG sites in the promotor region of RIIAD1 gene can be determined. In a further embodiment, when the methylation frequency obtained in the methylation analysis is more than or equal to a predetermined threshold, the presence of methylated CpG sites in the promotor region of RIIAD1 gene can be determined. The predetermined threshold is not particularly limited. The predetermined threshold may be empirically set based on accumulated data on various biological samples. The threshold may be alternatively set as follows. First, methylation frequency is analyzed for DNA extracted from a biological sample which is confirmed to be devoid of cancer cells derived from breast cancer (normal breast tissues or normal breast cells) and a biological sample containing a cancer cell derived from breast cancer. Next, based on the obtained analysis results, a threshold is set within a range that is higher than the methylation frequency of the biological sample devoid of cancer cells and lower than the methylation frequency of the biological sample containing the cancer cell. Preferably, the threshold is set as a value that can highly accurately differentiate between the biological sample devoid of cancer cells and the biological sample containing the cancer cell.

In the methods envisaged herein, information on breast cancer in a subject is obtained based on the analysis result obtained in the determining step. Obtaining information on gynecologic cancer in a subject can refer to the prediction, diagnosis, or prognosis of breast cancer in said subject. The information on breast cancer includes the presence or absence of cancer cells derived from breast cancer in a biological sample collected from a subject. In particular embodiments, the information on breast cancer may include information used as an index for diagnosis of breast cancer or information supporting diagnosis of breast cancer. Preferably, the information includes information indicating the occurrence of breast cancer, indicating that the status of breast cancer or information indicating both of them in a subject. The information includes, for example, the possibility of occurrence of breast cancer in a subject, or the risk for future occurrence of breast cancer in a subject. The information on breast cancer in a subject who has already been affected by breast cancer may include prognosis of the subject, or a degree of progression (stage).

In particular embodiments, when the result in the determining step indicates the presence of methylated CpG sites, information indicating the presence of cancer cells derived from breast cancer in a biological sample is obtained. Alternatively, information indicating the occurrence of breast cancer or indicating that the status of breast cancer is poor (or aggravated) can be obtained. Information indicating that a subject has a high risk for being affected by breast cancer or that a subject has already been affected by breast cancer can be obtained. For a subject who has already been affected by breast cancer, the information may indicate that prognosis of the subject is poor (or aggravated) or that the cancer is in a progressed stage.

In contrast, when the result in the determining step indicates the absence of methylated CpG sites, information indicating that a biological sample does not contain a cancer cell derived from breast cancer is obtained. Alternatively, information suggesting no occurrence of breast cancer or information indicating that breast cancer is in a preferable status can be obtained. The information may alternatively indicate that a subject has a low risk for becoming breast cancer or that a subject has not been affected by breast cancer. For a subject who has already been affected by breast cancer, the information may be indicative of a preferable prognosis of the subject or indicates that the cancer is in a relatively early stage.

The present disclosure also encompasses a marker for obtaining information on breast cancer by methylation analysis (hereinafter, simply referred to as "marker"). The marker envisaged herein is a nucleic acid which is isolated from the subject and includes at least one selected from CpG sites located in a promotor region of RIIAD1 gene. In the use of the marker envisaged herein, the methylation status of the marker is analyzed, and information on breast cancer in a subject can be obtained based on the analysis result.

The analysis of methylation status and the obtainment of information on breast cancer are the same as previously described.

Also disclosed herein is a nucleic acid derived from a nucleic acid isolated from a subject used as a marker for obtaining information on breast cancer. The nucleic acid derived from a nucleic acid isolated from a subject, which is used as such a maker, has a sequence obtained by subjecting a contiguous base sequence of the entire or partial promoter region of RIIAD1 gene to bisulfite treatment, and the promotor region includes a CpG site and a cytosine base not included in CpG sites. The cytosine not included in CpG sites may be any cytosine other than those contained in the CpG sites. Examples thereof include cytosines in the base sequence where cytosine (C) is adjacent to adenine (A), thymine (T) or cytosine (C) in this order from 5' to 3' (i.e., CA, CT, or CC). The nucleic acid can be obtained by subjecting a nucleic acid isolated from a subject to bisulfite treatment.

Regarding the nucleic acid used as the marker as envisaged herein, a non-methylated cytosine in the isolated DNA is converted to uracil by bisulfate treatment of the isolated DNA, while a methylated cytosine is not converted. In the methods provided herein, the information on breast cancer can be obtained by analyzing methylation status of CpG sites in the polynucleotide. The isolated DNA can be obtained in the same manner as that described for preparation of the DNA sample. The bisulfite treatment, the analysis of methylation status and the obtainment of information on breast cancer are also the same as previously described.

The size of the nucleic acid used as the marker as envisaged herein is not particularly limited as long as it allows analysis of methylation status by the MSP method, sequencing method or mass spectrometry, but is preferably 50 to 200 bases and more preferably 80 to 130 bases. Examples of the nucleic acid used as the marker as envisaged herein include a nucleic acid having a base sequence SEQ ID NO: 2. The nucleic acid having the base sequence SEQ ID NO: 2 is suitable for analysis of methylation status by the MSP method.

The present disclosure also encompasses a kit for detecting breast cancer (hereinafter, simply referred to as "kit"). The kit as envisaged herein includes a primer for amplifying a nucleic acid after bisulfite treatment. Such a primer is capable of specifically hybridizing to a nucleic acid consisting of a base sequence where cytosine present in a site other than a CpG site is converted to another base in a base sequence having a CpG site in a promotor region of RIIAD1 gene. Such a primer also specifically hybridizes to a region including a methylated CpG site. Accordingly, the primer may be capable of hybridizing specifically with nucleotide sequence comprising both a methylated CpG and a cytosine which is not in a CpG site and converted to another base.The kit may comprise a first primer which hybridizes specifically to a region including a methylated CpG site in a promotor region of RIIAD1 gene.

The primer included in the kits envisaged herein may be any primer for analysis of methylation status of CpG sites according to mass spectrometry such as MassARRAY (registered trademark) or an analysis method involving PCR amplification such as the MSP method and the bisulfite sequencing method. The primer is preferably a primer used for mass spectrometry such as Mass ARRAY (registered trademark) or for the MSP method. The base sequence of each primer may be appropriately selected by a person skilled in the art based on the base sequence in the promoter region. Examples of the primer include primers respectively having base sequences SEQ ID NOs: 3 and 4.

The kit may include a second primer. The second primer can hybridize to a nucleic acid consisting of a base sequence where cytosine is converted to another base in a base sequence having a CpG site in a promotor region of RIIAD1 gene and hybridize to a region including a sequence in which cytosine in a non-methylated CpG site is converted to another base by converting treatment.

The present disclosure also encompasses a computer program product for enabling a computer to provide information on breast cancer in a subject. Examples of the computer program product include a program downloadable from the Internet and a computer readable recording medium storing the program.

For example, there is a computer program for enabling the computer to carry out a process including the steps of:

obtaining the analysis result of methylation of a CpG site located in a promotor region of RIIAD1 gene included in a DNA sample derived from a subject from a measurement device; and

determining the presence or absence of breast cancer cells in the biological sample based on the obtained analysis result.

Hereinafter, an example of a suitable device for carrying out the method envisaged herein will be described with reference to the drawings. However, the present disclosure is not limited only to this example. Fig. 5 is a schematic view of an example of a diagnosis assisting device used for providing information on breast cancer in a subject. A diagnosis assisting device 10 illustrated in Fig. 5 includes a measurement device 20 and a determination device 30 connected to the measurement device 20.

The measurement device 20 may be, when methylation status is analyzed by the MSP method, a gel imaging device such as a fluorescence image scanner. In this case, the measurement device 20 onto which a gel obtained by electrophoresis of a reaction solution after nucleic acid amplification by the MSP method is mounted, detects amplification products. The measurement device 20 then obtains information on gel image of the amplification products and sends the obtained information to the determination device 30.

The measurement device 20 may be an MALDI-TOF mass spectrometer. In this case, the measurement device 20 obtains mass spectrometric information such as the time of flight or the mass-to-charge ratio (m/z value) of a substance to be analyzed. The measurement device 20, onto which a measurement sample prepared from a DNA sample derived from a subject is mounted, obtains mass spectrometric information on a nucleic acid in the measurement sample, and sends the obtained mass spectrometric information to the determination device 30.

The determination device 30 includes a computer main body 300, an input unit 301 including a keyboard or mouse, and a display unit 302 which is configured by an LCD or CRT and displays information on specimen or analysis result. The determination device 30 obtains information on gel image of the amplification products from the measurement device 20. The determination device 30 executes program to provide information on breast cancer in a subject based on these information. As shown in Fig. 5, the determination device 30 may be a device different from the measurement device 20, or may be a device that includes the measurement device 20. In the latter case, the determination device 30 may be the diagnosis assisting device 10 in itself.

Fig. 6 is a block diagram showing software of the computer main body 300 of the determination device 30 as a functional block. As shown in Fig. 6, the computer includes an acquisition unit 321, a storage unit 322, a calculating unit 323, a determination unit 324, and an output unit 325. The acquisition unit 321 is communicably connected to the measurement device 20 via a network.

The acquisition unit 321 obtains the information provided from the measurement device 20. The storage unit 322 stores a threshold necessary for determination, a formula for obtaining the band intensity, and a processing program. The calculating unit 323 calculates the band intensity according to the stored processing program using the information obtained by the acquisition unit 321. The determination unit 324 determines whether the band intensity which has been obtained by the acquisition unit 321 or calculated by the calculating unit 323 is not less than the threshold stored in the storage unit 322. The output unit 325 outputs the analysis result determined by the determination unit 324 to the display unit 302 as information on breast cancer in a subject (e.g., the presence or absence of breast cancer cells in a biological sample collected from a subject).

Fig. 7 is a block diagram showing a hardware configuration of the computer main body 300 shown in Fig. 6. As shown in Fig. 7, the computer main body 300 includes a central processing unit (CPU) 310, a read only memory (ROM) 311, a random access memory (RAM) 312, a hard disk 313, an input/output interface 314, a readout device 315, a communication interface 316, and an image output interface 317. The CPU 310, the ROM 311, the RAM 312, the hard disk 313, the input/output interface 314, the readout device 315, the communication interface 316, and the image output interface 317 are data-communicably connected by a bus 318.

The CPU 310 can execute the program stored in the ROM 311 and the program loaded on the RAM 312. When the CPU 310 executes the program, each of the functions shown in Fig. 6 is executed. As a result, the determination device 30 functions as the determination device for providing information on breast cancer in a subject.

The ROM 311 is configured to include mask ROM, PROM, EPROM, EEPROM, and the like. As described above, the ROM 311 stores program to be executed by the CPU 310 and data used for the program.

The RAM 312 is configured to include SRAM and DRAM. The RAM 312 is used to read out the programs recorded on the ROM 311 and the hard disk 313. The RAM 312 is also used as a working area of the CPU 310 when these programs are executed.

The hard disk 313 is installed with programs to be executed by the CPU 310, such as operating system and application program (computer program for providing information on breast cancer in a subject) as well as data used in executing the programs.

The readout device 315 is configured to include a flexible disk drive, a CD-ROM drive, and a DVD-ROM drive. The readout device 315 can read out programs or data recorded on a portable recording medium 40.

The input/output interface 314 is configured to include a serial interface such as USB, IEEE 1394 or RS-232C, a parallel interface such as SCSI, IDE or IEEE 1284, and an analog interface including a D/A converter or an A/D converter. The input unit 301 such as a keyboard or mouse is connected to the input/output interface 314. An operator can input various instructions to the computer main body 300 via the input unit 301.

The communication interface 316 is, for example, an Ethernet (registered trademark) interface. The computer main body 300 can send printing data to a printer and the like via the communication interface 316.

The image output interface 317 is connected to the display unit 302 including LCD, CRT or the like. Thus, the display unit 302 can output a video signal according to the image data provided from the CPU 310. The display unit 302 displays the image (screen) according to the input video signal.

Subsequently, the procedure of obtaining information on breast cancer in a subject which is processed by the diagnosis assisting device 10 will be described. Fig. 8 is an example of a flow chart for obtaining information on breast cancer. The case of obtaining the band intensity from the information on gel image obtained using the biological sample from the subject and determining whether the obtained band intensity is not less than the threshold will be described as an example. In step S1-1, the acquisition unit 321 of the diagnosis assisting device 10 obtains information on gel image of RIIAD1 gene from the measurement device 20. In step S1-2, the calculating unit 323 obtains the band intensity from the obtained information on gel image. The calculating unit 323 sends the information to the storage unit 322.

Thereafter, in step S1-3, the determination unit 324 determines whether the band intensity obtained in step S1-2 is not less than the threshold stored in the storage unit 322. When the band intensity is higher than or equal to the threshold, the routine proceeds to step S1-4. Then, the determination unit 324 sends the analysis result showing that breast cancer cells are present in the biological sample from the subject to the output unit 325. On the other hand, when the band intensity is lower than the threshold, the routine proceeds to step S1-5. Then, the determination unit 324 sends the analysis result showing that breast cancer cells are not present in the biological sample from the subject to the output unit 325.

In step S1-6, the output unit 325 finally outputs the analysis result as the information on breast cancer in a subject. The output unit 325 displays it on the display unit 302. Consequently, the diagnosis assisting device 10 can provide a physician or the like information assisting to judge whether the subject has breast cancer.

Hereinafter, the present disclosure will be described in detail with reference to examples, however the present disclosure is not limited to the examples.

## EXAMPLES

### Example 1:

Identification of Novel Markers Utilizing Methylation Data of Cancerous Tissues and Non-Cancerous Tissues of Breast Cancer, and Normal Mammary Tissues

(1) Collection of Methylation Data

In Example 1, Methylation data on Infinium HumanMethylation450 BeadChip (Illumina, Inc.), which are published in TCGA (The Cancer Genome Atlas: http://tcga-data.nci.nih.gov/tcga/tcgaHome2.jsp), were collected for cancerous tissues (548 specimens) and non-cancerous (mammary) tissues (98 specimens) of breast cancer. Further, methylation data on Infinium HumanMethylation450 BeadChip, which are published in the publication of Nazor K L et al. (Recurrent variations in DNA methylation in human pluripotent stem cells and their differentiated derivatives. Cell Stem Cell 2012; 10(5): 620-634), were collected for normal mammary tissues (23 specimens).

(2) Identification of Novel Markers

As a result of data mining using Infinium HumanMethylation450 BeadChip (Illumina, Inc.), the promotor region of RIIAD1 gene was identified as a marker which is specifically methylated in cancerous tissues of breast cancer (refer to Fig. 1). These markers are also referred to as "present markers" hereinbelow.

### Example 2:

Comparison of Methylation Data between Cancer Derived from Plural Types of Cancer/Cancer from Tumor/Tumor Tissue Specimens, Non-Cancerous Tissue Specimens, and Normal Tissue Specimens

(1) Collection of Methylation Data

In Example 2, methylation data of 15 types of cancer/tumor tissue specimens, 12 types of non-cancerous tissue specimens, and 19 types of normal tissue specimens were compared. The number of specimens for the respective tissues is shown in the following tables.

[Table 2]

| Cancer/tumor specimens | |
|---|---|
| Tissues | The number of specimens |
| Brain tumor | 114 |
| Lung cancer | 370 |
| Breast cancer | 548 |
| Gastric cancer | 69 |
| Colon cancer | 324 |
| Liver cancer | 81 |
| Renal cancer | 363 |
| Bladder cancer | 78 |
| Uterine body cancer | 334 |
| Ovarian cancer | 49 |
| Prostatic cancer | 153 |

(continued)

| Cancer/tumor specimens | |
|---|---|
| Tissues | The number of specimens |
| Acute leukemia | 192 |
| Sarcoma | 73 |
| Malignant melanoma | 242 |
| Thyroid cancer | 316 |

[Table 3]

| Non-cancerous tissue | |
|---|---|
| Tissues | The number of specimens |
| Brain tumor | 1 |
| Lung cancer | 75 |
| Breast cancer | 98 |
| Gastric cancer | 2 |
| Colon cancer | 40 |
| Hepatic cancer | 14 |
| Renal cancer | 208 |
| Bladder cancer | 15 |
| Uterine body cancer | 36 |
| Ovarian cancer | 1 |
| Prostatic cancer | 49 |
| Thyroid cancer | 36 |

[Table 4]

| Normal tissues | | | | |
|---|---|---|---|---|
| Tissues | RCAST | Literature 1 | Literature 2 | Total |
| Normal brain (Brain) | 2 | 1 | 0 | 3 |
| Normal oral cavity (Oral) | 2 | 0 | 0 | 2 |
| Normal lung (Lung) | 0 | 2 | 0 | 2 |
| Normal colonic mucosa (Colon) | 2 | 0 | 0 | 2 |
| Normal liver (Liver) | 2 | 0 | 0 | 2 |
| Peripheral blood from healthy subjects (Blood) | 2 | 2 | 0 | 4 |
| Normal skeletal muscle (Skeletal) | 2 | 2 | 0 | 4 |
| Normal testis (Testis) | 1 | 0 | 0 | 1 |
| Normal gastric mucosa (Stomach) | 0 | 1 | 0 | 1 |
| Normal pancreas (Pancreas) | 0 | 2 | 0 | 2 |
| Normal spleen (Spleen) | 0 | 2 | 0 | 2 |
| Normal kidney (Kidney) | 0 | 0 | 0 | 0 |

(continued)

| Normal tissues | | | | |
|---|---|---|---|---|
| Tissues | RCAST | Literature 1 | Literature 2 | Total |
| Normal adrenal gland (Adrenal gland) | 0 | 2 | 0 | 2 |
| Normal ureter (Ureter) | 0 | 2 | 0 | 2 |
| Normal bladder (Bladder) | 0 | 2 | 0 | 2 |
| Normal lymph nodes (Lymph nodes) | 0 | 2 | 0 | 2 |
| Normal adipose tissue (Adipose tissue) | 0 | 2 | 0 | 2 |
| Normal heart (Heart) | 0 | 1 | 0 | 1 |
| Various normal blood cell components (WB, PBMC, Gran, CD4+, CD8+, CD14+, CD19+, CD56+, Neu, Eos) | 0 | 0 | 60 | 60 |

In Table 4, the methylation data for the specimens indicated in the column "RCAST" were obtained by the present inventors according to Infinium Methylation Assay using Infinium HumanMethylation450 BeadChip (Illumina, Inc.). The methylation data for the specimens indicated in the columns "Literature 1" and "Literature 2" were methylation data published in the following literatures obtained with Infinium HumanMethylation450 BeadChip (Illumina, Inc.).

Literature 1: Nazor KL et al., Recurrent variations in DNA methylation in human pluripotent stem cells and their differentiated derivatives. Cell Stem Cell 2012; 10(5): 620-634
Literature 2: Reinius LE et al., Differential DNA Methylation in Purified Human Blood Cells: Implications for Cell Lineage and Studies on Disease Susceptibility, PLoS One, 7(7) e41361

The methylation data in this context are the methylation rate (mCpG) of a CpG site in RIIAD1 obtained as follows. The Infinium HumanMethylation450 BeadChip include probes for methylated CpG sites and probes for non-methylated CpG sites of 482,421 CpG sites on human genome. The signal intensity (signal M) from the probes for methylated CpG sites and the signal intensity (signal U) from the probes for non-methylated CpG sites in the target genes were detected on Bead Array Reader, and the methylation rate (mCpG) of CpG sites in the respective genes was calculated according to the following calculation formula:

$$(\text{mCpG}) = (\text{signal M})/\{(\text{signal M}) + (\text{signal U})\}$$

(2) Comparison of Methylation Positive Rates between Cancer/Tumor Tissue Specimens, Non-Cancerous Tissue Specimens, and Normal Tissue Specimens

The obtained methylation rate (mCpG) was defined as "methylation positive" when a statistically significant difference between tumor tissue specimens and normal tissue specimens was observed. Then, the methylation positive rate (%) for each cancer was calculated according to the following formula:

$$\text{Methylation positive rate (\%)} = (\text{the number of methylation positive specimens/the total number of specimens}) \times 100$$

(For example, for brain tumor, the methylation positive rate was calculated by "(the number of methylation positive specimens among the brain tumor tissue specimens/the total number of the brain tumor tissue specimens = 114) × 100.")

The results are shown in Fig. 2. In Fig. 2, "normal tissues" represent, among the tissues indicated in Table 4, normal tissues excluding 60 specimens of various normal blood cell components, and "normal blood cells" represent the 60 specimens of various normal blood cell components. As obvious from Fig. 2, all the present markers were rarely methylated in non-cancerous tissues, human normal tissues, and human normal blood cells. Further, all the present markers are specifically highly methylated in breast cancer, compared to other types of cancer. Therefore, it is shown that the present markers are suitable for detection of breast cancer.

Example 3:

Comparison of Methylation Data between Tissues from Healthy Subjects and Tissues from Breast Cancer Patients

(1) Biological Samples

In Example 3, as biological samples derived from breast cancer patients, cancerous tissues collected from breast cancer patients (3 specimens) were used. As control samples, normal mammary tissues (3 specimens) were used.

(2) Preparation of Measurement Samples

(i) Extraction of Genomic DNA

Genomic DNA was extracted from the tissue samples with the use of QIAamp DNA Mini Kit (QIAGEN). Genomic DNA of human peripheral blood lymphocytes was used as the control genomic DNA. The genomic DNA from human peripheral blood lymphocytes was amplified with the use of GenomiPhi v2DNA Amplification Kit (GE Healthcare Life Sciences). The obtained amplified product consisted of non-methylated DNA. The amplification product was fragmented with Bioruptor (COSMO BIO Co., Ltd.) to obtain a solution of non-methylated DNA fragments (0% methylated DNA). A portion of the solution of non-methylated DNA fragments was subjected to reaction with SssI methylase (New England Biolabs) to methylate all cytosines in CG sequences, and a solution of methylated DNA fragments (100% methylated DNA) was obtained.

(ii) Bisulfite Treatment

The respective DNA fragments (500 ng) obtained as described above were subjected to bisulfite treatment with the use of EZ DNA Methylation Kit (Zymo Research), and the treated genomic DNA was dissolved in sterilized distilled water (80 μl).

(3) Methylation-Specific PCR (MSP)

MSP was carried out using the measurement samples and control samples obtained in the above section (2). The composition of the PCR reagent, primer sets, and reaction conditions for PCR are shown below.

<PCR reagent>

| | |
|---|---|
| DW (sterilized water) | 18.8 μL |
| 10 × PCR buffer with MgCl2 (Roche) | 2.5 μL |
| 10 mM dNTP mix | 0.5 μL |
| 10 μM sense primer | 1.0 μL |
| 10 μM antisense primer | 1.0 μL |
| Faststart Taq polymerase (Roche) | 0.2 μL |
| Measurement sample | 1.0 μL |
| Total | 25.0 μL |

<Primer Set>

The primer sets used for MSP are shown in Table 5. These primer sets allow generation of amplification products when DNA in the amplified target regions is methylated (hereinafter also referred to as "primer set for methylation detection"). As a primer set for accuracy control, a primer set that allows determination on whether or not the bisulfite treatment has been appropriately performed (refer to Table 6). The base sequence of region which is analyzed with the primer set for methylation detection in the promoter region of RIIAD1 gene is shown in SEQ ID NO: 7. The base sequence obtained by bisulfite conversion of this region is shown in SEQ ID NO: 2.

[Table 5]

| Gene symbol | Base sequence | SEQ ID NO: | PCR product (bp) | Amplified region |
|---|---|---|---|---|
| *RIIAD1* | CGATCGTAGTAAGATGGAGACGT | 3 | 133 | chr1: 151, 694, 000-151, 694, 132 |
| | TTAATAAACGCTAAATAAAAACGCG | 4 | | |

[Table 6]

| Primer for accuracy control | Base sequence | SEQ ID NO: | PCR product (bp) |
|---|---|---|---|
| Forward | GGGATATTAAGTGGAGTTATTTTGGTTTTAGTT | 5 | 129 |
| Reverse | CCCTCCCAACATCCTTCCTAA | 6 | |

<PCR Reaction Conditions>

95°C for 6 minutes; X cycles of 95°C for 30 seconds, 60°C for 15 seconds, and 72°C for 30 seconds; 72°C for 7 minutes; and keep at 16°C

The number of cycles "X" is 38 for the primer set for methylation detection, and 40 for the primer set for accuracy control.

(4) Result Analysis

The amplified product obtained from MSP was verified by 2% agarose gel electrophoresis. The results are shown in Fig. 3. In this figure, "0" and "100" under "control" represent the 0% methylation control sample and the 100% methylation control sample, respectively.

In PCR using the primer set for accuracy control, bands were detected for all the samples as shown in Fig. 3. This shows that bisulfite treatment of the samples was appropriately carried out. In PCR using the primer sets for samples of methylation detection, bands derived from methylated CpGs were not detected for any normal mammary tissues. On the other hand, bands were detected in two cases of three cases for samples of mammary tissues. Accordingly, it turns out that in methylation analysis of the present markers by the MSP method, the methylation of the present markers and breast cancer were correlated similarly to the result from Infinium method of Example 1. Specifically, it turns out that RIIAD1 is a highly specific marker which is highly methylated in breast cancer, but methylation of which is not detected in normal mammary tissues.

Example 4:

Comparison of Methylation Data between Various Cancer Tissues and Normal Tissues

(1) Biological Samples

In Example 4, as biological samples, tissue samples collected from various cancer patients and normal tissues from various organs were used. Details of each sample are shown in Table 7. In Table 7, "T" represents tumor tissue, "N" represents normal tissue, and "PBL" represents peripheral blood leukocyte.

[Table 7]

| Derived tissue | | The number of specimens | Donor | Notation |
|---|---|---|---|---|
| Colon | Cancer | 2 | Purchased from BioChain Institute, Inc. | T1 |
| | | | | T2 |
| | Normal | 3 | | N1 |
| | | | | N2 |
| | | | | N3 |
| Brain | Cancer | 1 | The University of Tokyo | T |
| | Normal | 2 | Purchased from BioChain Institute, Inc. | N1 |
| | | | | N2 |
| Lung | Cancer | 1 | Purchased from BioChain Institute, Inc. | T |
| | Normal | 2 | The University of Tokyo | N1 |
| | | | | N2 |
| Liver | Cancer | 1 | Purchased from BioChain Institute, Inc. | T |
| | Normal | 2 | The University of Tokyo | N1 |
| | | | | N2 |
| Kidney | Cancer | 1 | Purchased in BioChain Institute, Inc. | T |
| | Normal | 2 | The University of Tokyo | N1 |
| | | | | N2 |
| Uterine body | Cancer | 1 | The University of Tokyo | T |
| | Normal | 2 | | N1 |
| | | | | N2 |
| Bladder | Cancer | 1 | Purchased from BioChain Institute, Inc. | T |
| | Normal | 1 | | N |
| Cervix uteri | Normal | 1 | Purchased from BioChain Institute, Inc. | Cervix |
| Testis | Normal | 1 | Purchased from BioChain Institute, Inc. | Testis |
| Blood cell | Normal | 1 | Purchased from BioChain Institute, Inc. | PBL |
| Stomach | Cancer | 1 | Purchased from BioChain Institute, Inc. | T |
| | Normal | 1 | | N |

(2) Preparation of Measurement Samples and Control Samples

(i) Extraction of Genomic DNA

Genomic DNA was extracted from the tissue samples in the same manner as in Example 3. Genomic DNA of human peripheral blood lymphocytes was used as the control genomic DNA. A solution of non-methylated DNA fragments (0% methylated DNA) and a solution of methylated DNA fragments (100% methylated DNA) were prepared from the genomic DNA of human peripheral blood lymphocytes in the same manner as in Example 3.

(ii) Bisulfite Treatment

The respective DNA fragments (500 ng) obtained as described above were subjected to bisulfite treatment with the use of EZ DNA Methylation Kit (Zymo Research), and the treated genomic DNA was dissolved in sterilized distilled water (80 μl).

(3) MSP

MSP was carried out using the measurement samples and control samples (DNA after bisulfite treatment) obtained in the above section (2). The composition of the PCR reagent, primer sets, and reaction conditions for PCR used for MSP in Example 4 are the same as those in Example 3.

(4) Result Analysis

The amplified product obtained from MSP was verified by 2% agarose gel electrophoresis. The results are shown in Fig. 4. In this figure, "NC" and "PC" represent the 0% methylation control sample and the 100% methylation control sample, respectively. "Accuracy control" represents the amplification product of the primer for accuracy control.

In PCR using the primer set for accuracy control, bands were detected for all the samples as shown in Fig. 4. Thus, this shows that bisulfite treatment of the samples was appropriately carried out. In PCR using the primer sets for methylation detection, bands derived from methylated CpGs were not detected for any cancer tissues and normal tissues. This result suggests that the present markers are specific for breast cancer.

## Claims

**1.** 1. A method of obtaining information on breast cancer comprising the steps of:

analyzing methylation status of a CpG site located in a promoter region of RIIAD1 gene in the DNA sample prepared from a biological sample collected from a subject; and
obtaining information indicating the presence of breast cancer cells in the biological sample when the result in the determining step indicates the presence of a methylated CpG site.

2. The method according to claim 1, wherein the methylation status of SEQ ID NO: 7 is analyzed in the analyzing step.

3. The method according to claim 1 or 2, wherein the analyzing step is conducted by at least one method selected from the group consisting of mass spectrometry and methylation-specific PCR method.

4. The method according to any one of claims 1 to 3, wherein the biological sample is a tissue, blood or serum.

5. The method according to any one of claims 1 to 4, wherein
the analyzing step comprises: calculating methylation frequency of the promoter region, and
the obtaining step comprises: comparing the frequency with a predetermined threshold; and obtaining information indicating the presence of breast cancer cells in the biological sample when the frequency is equal to or higher than the threshold.

6. The method according to any one of claims 1 to 5, wherein said method comprises preparing a DNA sample from the biological sample.

7. The method according to claim 6, wherein the preparing step comprises treating DNA in the biological sample with bisulfite.

8. Use of a reagent kit for detection of breast cancer, wherein the reagent kit comprises a primer that hybridizes to a promotor region of RIIAD1 gene, wherein the promoter region comprises: a uracil base converted from unmethylated cytosine base by a bisulfite treatment; and methylated cytosine base, and wherein the primer is used for analyzing methylation status of a CpG site in the promoter region of RIIAD1 gene in a DNA sample.

9. Use according to claim 8, wherein the primer comprises a base sequence SEQ ID NO: 3 or 4.

**Patentansprüche**

1. Verfahren zur Gewinnung von Informationen über Brustkrebs, umfassend die Schritte:

   Analysieren des Methylierungsstatus einer in einer Promotorregion des RIIAD1-Gens liegenden CpG-Stelle in der aus einer einem Individuum entnommenen biologischen Probe hergestellten DNA-Probe; und
   Gewinnen von Informationen, die das Vorliegen von Brustkrebszellen in der biologischen Probe anzeigen, wenn das Ergebnis im Bestimmungsschritt das Vorliegen einer methylierten CpG-Stelle anzeigt.

2. Verfahren nach Anspruch 1, wobei im Analyseschritt der Methylierungsstatus von SEQ ID NO: 7 analysiert wird.

3. Verfahren nach Anspruch 1 oder 2, wobei der Analyseschritt mit wenigstens einem aus der aus Massenspektrometrie und methylierungsspezifischem PCR-Verfahren bestehenden Gruppe ausgewählten Verfahren ausgeführt wird.

4. Verfahren nach einem der Ansprüche 1 bis 3, wobei es sich bei der biologischen Probe um ein Gewebe, Blut oder Serum handelt.

5. Verfahren nach einem der Ansprüche 1 bis 4, wobei der Analyseschritt Berechnen der Methylierungshäufigkeit der Promotorregion umfasst und
der Gewinnungsschritt Vergleichen der Häufigkeit mit einem vorbestimmten Schwellenwert; und Gewinnen von Informationen, die das Vorliegen von Brustkrebszellen in der biologischen Probe anzeigen, wenn die Häufigkeit gleich dem oder höher als der Schwellenwert ist, umfasst.

6. Verfahren nach einem der Ansprüche 1 bis 5, wobei das Verfahren Herstellen einer DNA-Probe aus der biologischen Probe umfasst.

7. Verfahren nach Anspruch 6, wobei der Herstellungsschritt Behandeln von DNA in der biologischen Probe mit Bisulfit umfasst.

8. Verwendung eines Reagentienkits zum Nachweis von Brustkrebs, wobei das Reagentienkit einen Primer umfasst, der an eine Promotorregion des RIIAD1-Gens hybridisiert, wobei die Promotorregion eine durch eine Bisulfitbehandlung aus unmethyliertem Cytosin überführte Uracilbase; und methylierte Cytosinbase umfasst, und wobei der Primer zum Analysieren des Methylierungsstatus einer CpG-Stelle, in der Promotorregion des RIIAD1-Gens in einer DNA-Probe verwendet wird.

**9.** Verwendung nach Anspruch 8, wobei der Primer eine Basensequenz SEQ ID NO: 3 oder 4 umfasst.

## Revendications

**1.** Méthode d'obtention d'informations sur le cancer du sein comprenant les étapes suivantes :

analyse du statut de méthylation d'un site CpG situé dans une région de promoteur du gène RIIAD1 dans l'échantillon d'ADN préparé à partir d'un échantillon biologique collecté auprès d'un sujet ; et
obtention d'informations indiquant la présence de cellules de cancer du sein dans l'échantillon biologique lorsque le résultat de l'étape déterminante indique la présence d'un site CpG méthylé.

**2.** Méthode selon la revendication 1, où le statut de méthylation de la SEQ ID NO: 7 est analysé dans l'étape d'analyse.

**3.** Méthode selon la revendication 1 ou 2, où l'étape d'analyse est mise en oeuvre par au moins une méthode choisie dans le groupe constitué par la spectrométrie de masse et la méthode de PCR spécifique de la méthylation.

**4.** Méthode selon l'une quelconque des revendications 1 à 3, où l'échantillon biologique est un échantillon de tissu, de sang ou de sérum.

**5.** Méthode selon l'une quelconque des revendications 1 à 4, où
l'étape d'analyse comprend : le calcul de la fréquence de méthylation de la région de promoteur, et
l'étape d'obtention comprend : la comparaison de la fréquence à un seuil prédéterminé ; et l'obtention d'informations indiquant la présence de cellules de cancer du sein dans l'échantillon biologique lorsque la fréquence est supérieure ou égale au seuil.

**6.** Méthode selon l'une quelconque des revendications 1 à 5, où ladite méthode comprend la préparation d'un échantillon d'ADN à partir de l'échantillon biologique.

**7.** Méthode selon la revendication 6, où l'étape de préparation comprend le traitement de l'ADN dans l'échantillon biologique par du bisulfite.

**8.** Utilisation d'un kit de réactifs pour la détection du cancer du sein, où le kit de réactifs comprend une amorce qui s'hybride à une région de promoteur du gène RIIAD1, où la région de promoteur comprend : une base uracile convertie à partir d'une base cytosine non méthylée par un traitement par bisulfite ; et une base cytosine méthylée, et où l'amorce est utilisée pour l'analyse du statut de méthylation d'un site CpG dans la région de promoteur du gène RIIAD1 dans un échantillon d'ADN.

**9.** Utilisation selon la revendication 8, où l'amorce comprend une séquence de bases SEQ ID NO: 3 ou 4.

FIG. 1

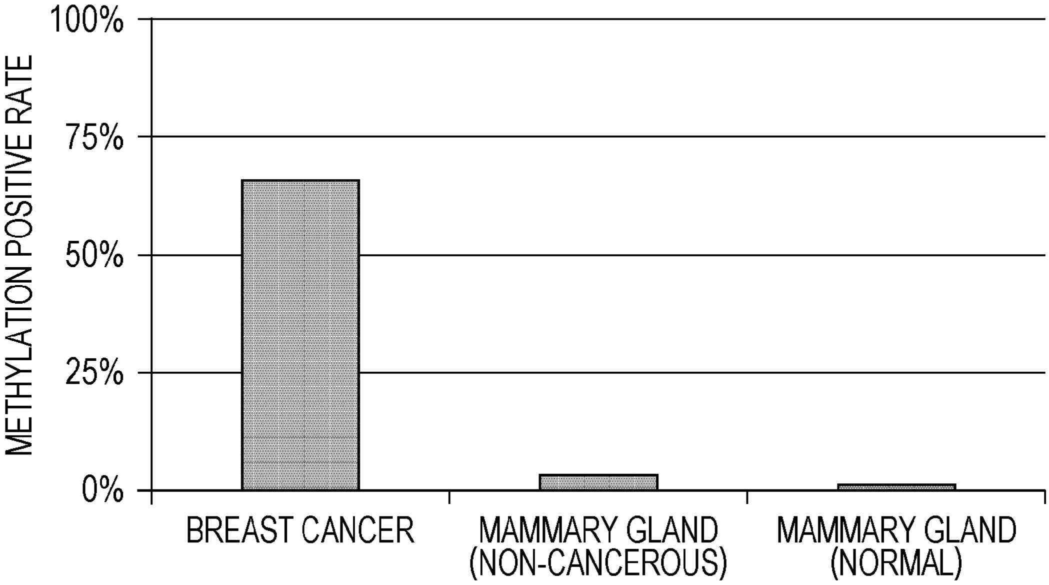
METHYLATION POSITIVE RATE
100%
75%
50%
25%
0%
BREAST CANCER
MAMMARY GLAND (NON-CANCEROUS)
MAMMARY GLAND (NORMAL)

FIG. 2

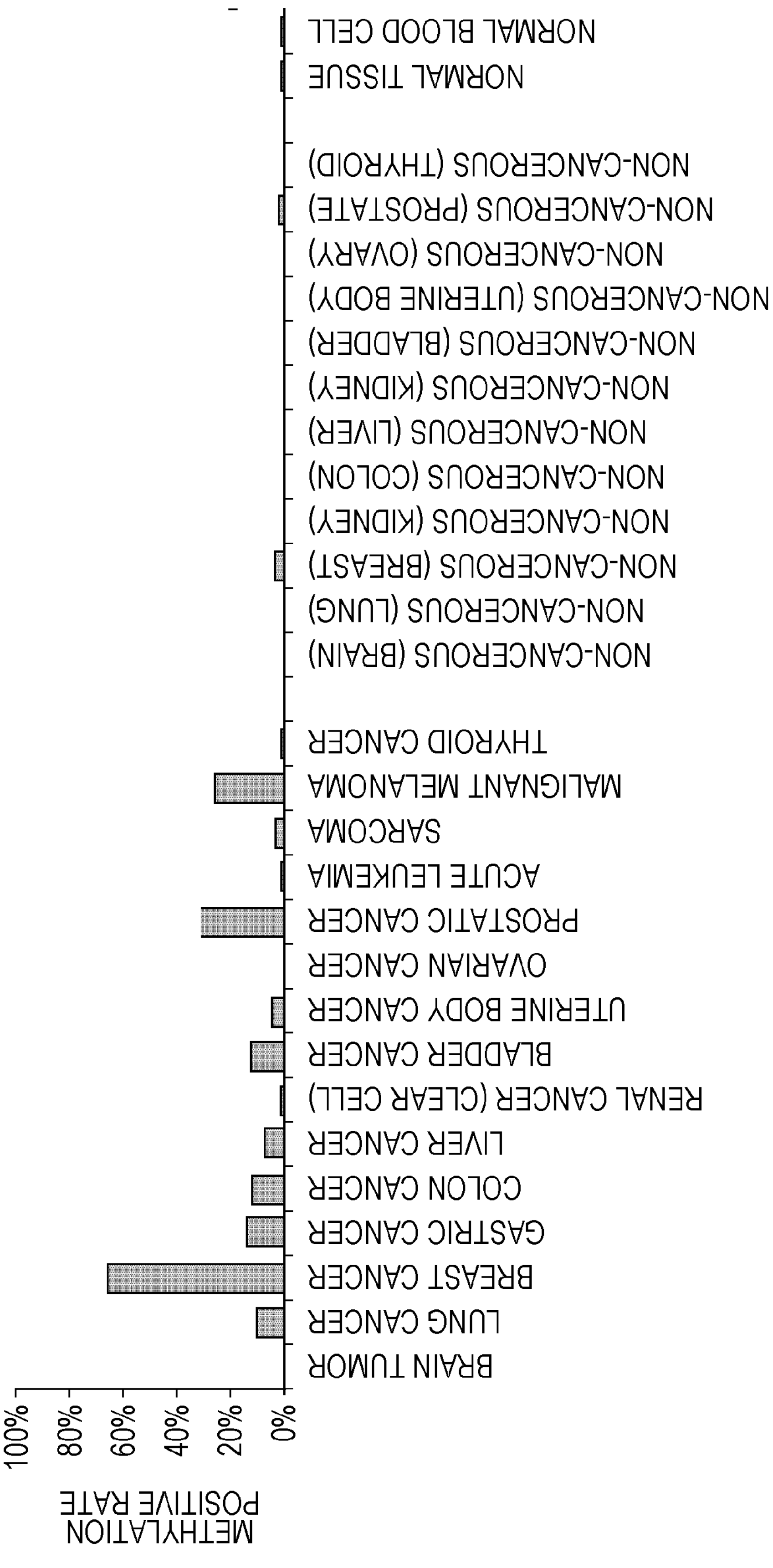
METHYLATION POSITIVE RATE
100%
80%
60%
40%
20%
0%
BRAIN TUMOR
LUNG CANCER
BREAST CANCER
GASTRIC CANCER
COLON CANCER
LIVER CANCER
RENAL CANCER (CLEAR CELL)
BLADDER CANCER
UTERINE BODY CANCER
OVARIAN CANCER
PROSTATIC CANCER
ACUTE LEUKEMIA
SARCOMA
MALIGNANT MELANOMA
THYROID CANCER
NON-CANCEROUS (BRAIN)
NON-CANCEROUS (LUNG)
NON-CANCEROUS (BREAST)
NON-CANCEROUS (KIDNEY)
NON-CANCEROUS (COLON)
NON-CANCEROUS (LIVER)
NON-CANCEROUS (KIDNEY)
NON-CANCEROUS (BLADDER)
NON-CANCEROUS (UTERINE BODY)
NON-CANCEROUS (OVARY)
NON-CANCEROUS (PROSTATE)
NON-CANCEROUS (THYROID)
NORMAL TISSUE
NORMAL BLOOD CELL

FIG. 3

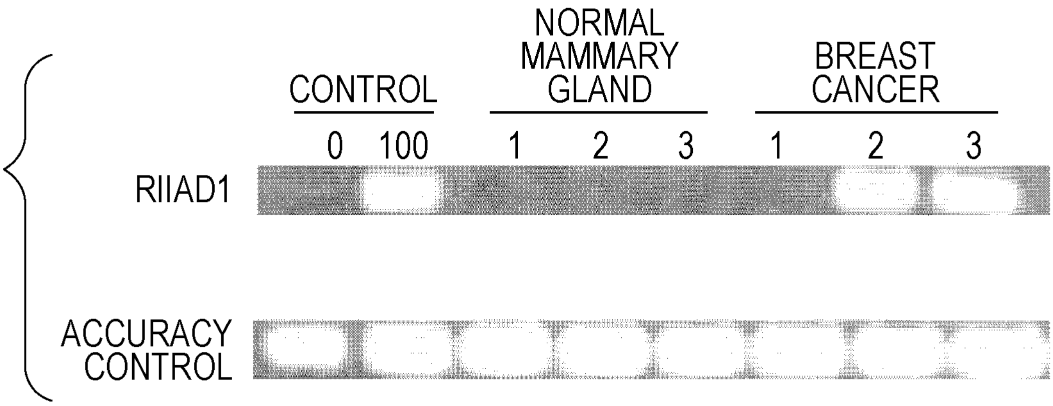
CONTROL
NORMAL MAMMARY GLAND
BREAST CANCER
0
100
1
2
3
1
2
3
RIIAD1
ACCURACY CONTROL

FIG. 4

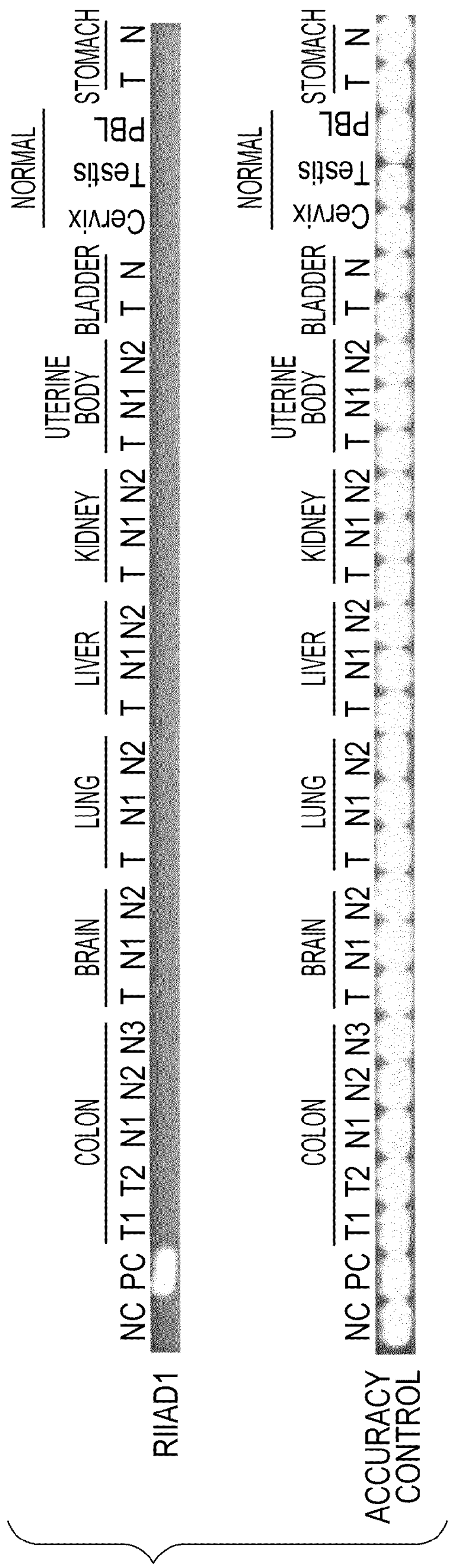
RIIAD1
ACCURACY CONTROL
NC PC
COLON
T1 T2 N1 N2 N3
BRAIN
T N1 N2
LUNG
T N1 N2
LIVER
T N1 N2
KIDNEY
T N1 N2
UTERINE BODY
T N1 N2
BLADDER
T N
NORMAL
Cervix Testis PBL
STOMACH
T N

FIG. 5

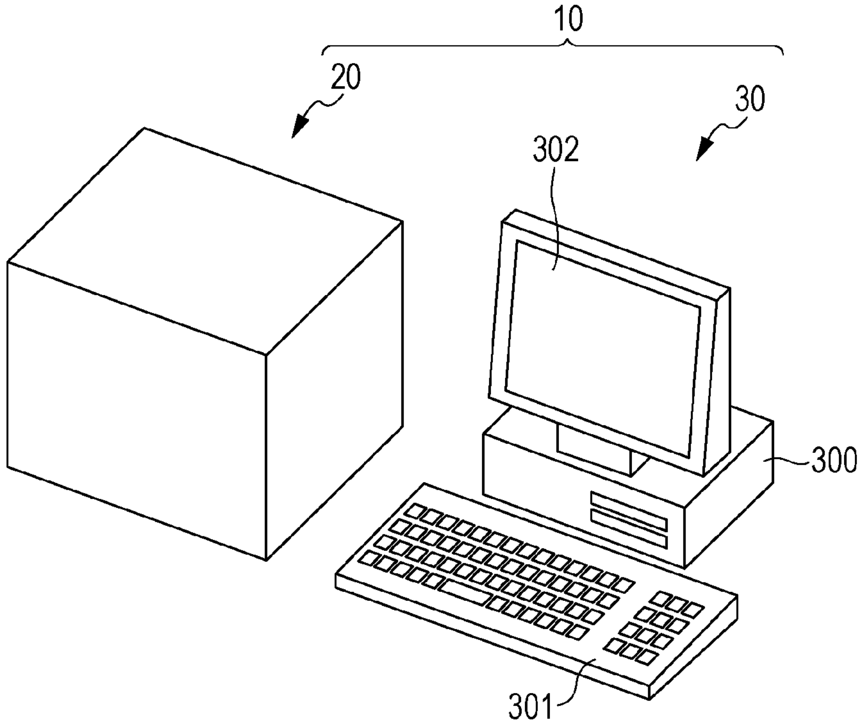
10
20
30
302
300
301

FIG. 6

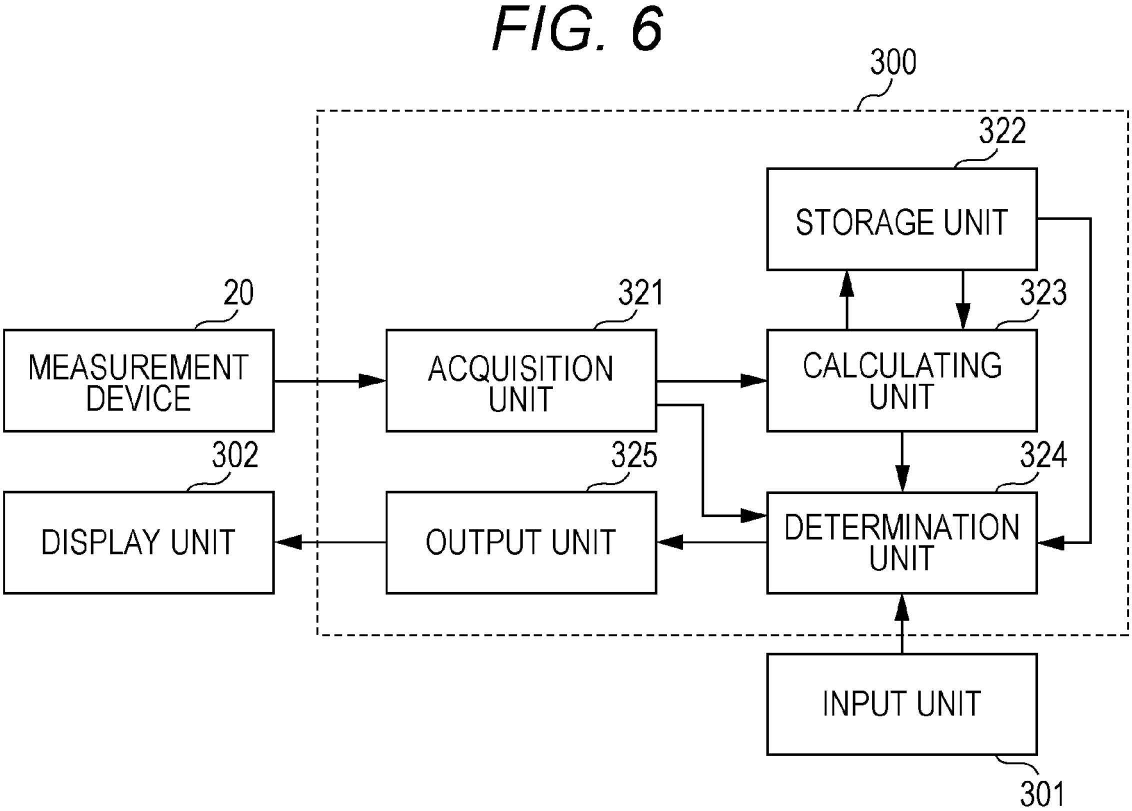
300
322
STORAGE UNIT
20
321
323
MEASUREMENT DEVICE
ACQUISITION UNIT
CALCULATING UNIT
302
325
324
DISPLAY UNIT
OUTPUT UNIT
DETERMINATION UNIT
INPUT UNIT
301

FIG. 7

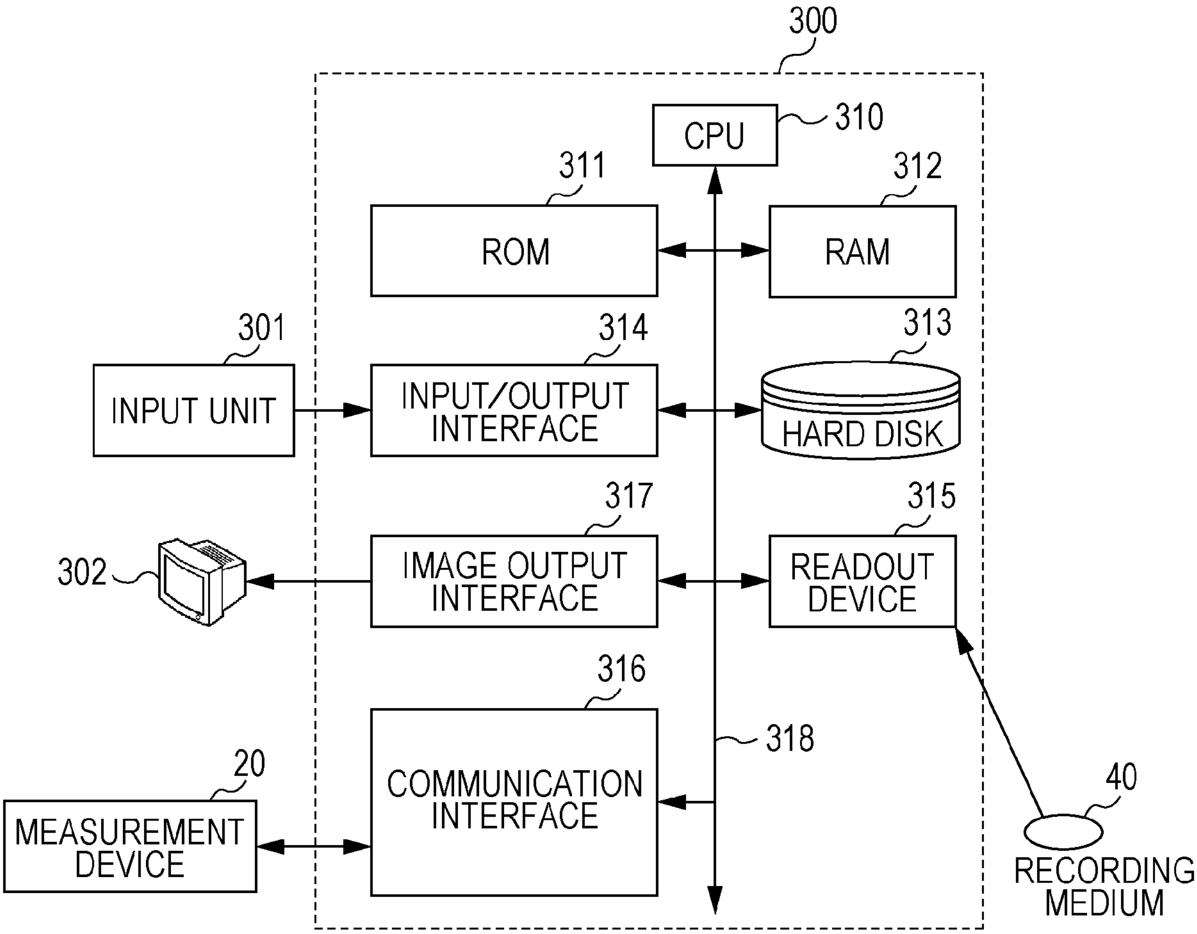
300
CPU
310
311
312
ROM
RAM
301
314
313
INPUT UNIT
INPUT/OUTPUT INTERFACE
HARD DISK
317
315
302
IMAGE OUTPUT INTERFACE
READOUT DEVICE
316
318
20
40
MEASUREMENT DEVICE
COMMUNICATION INTERFACE
RECORDING MEDIUM

FIG. 8

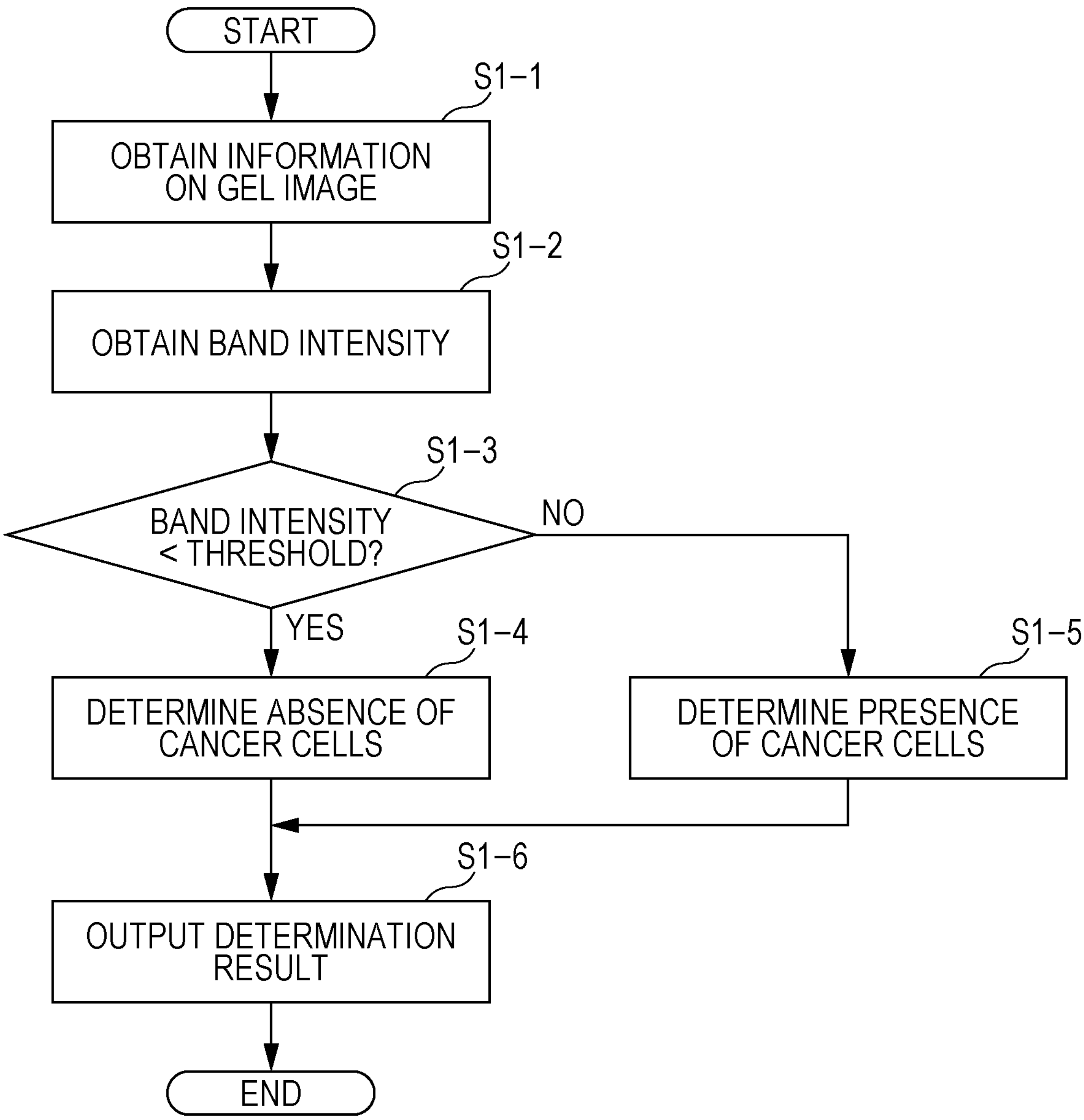
START
S1-1
OBTAIN INFORMATION ON GEL IMAGE
S1-2
OBTAIN BAND INTENSITY
S1-3
BAND INTENSITY < THRESHOLD?
NO
YES
S1-4
DETERMINE ABSENCE OF CANCER CELLS
S1-5
DETERMINE PRESENCE OF CANCER CELLS
S1-6
OUTPUT DETERMINATION RESULT
END

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2011318738 A
- EP 2341150 A
- JP 2015073577 A


### Non-patent literature cited in the description


- Tumor Suppressor Gene Promoter Hypermethylation in Serum of Breast Cancer Patients. **DULAIMI E. et al.** Clin Cancer Res. 2004, vol. 10, 6189-6193
- **DULAIMI E. et al.** Tumor Suppressor Gene Promoter Hypermethylation in Serum of Breast Cancer Patients. *Clin Cancer Res,* 2004, vol. 10, 6189-6193
- **NAZOR K L et al.** Recurrent variations in DNA methylation in human pluripotent stem cells and their differentiated derivatives. *Cell Stem Cell,* 2012, vol. 10 (5), 620-634
- **NAZOR KL et al.** Recurrent variations in DNA methylation in human pluripotent stem cells and their differentiated derivatives. *Cell Stem Cell,* 2012, vol. 10 (5), 620-634
- **REINIUS LE et al.** Differential DNA Methylation in Purified Human Blood Cells: Implications for Cell Lineage and Studies on Disease Susceptibility. *PLoS One,* vol. 7 (7), e41361

SEQUENCE LISTING

<110> THE UNIVERSITY OF TOKYO SYSMEX CORPORATION

<120> Method for obtaining information on breast cancer and kit for detecting breast cancer

<130> SYSM-106-EP

<150> JP 2015-073577
<151> 2015-03-31

<160> 7

<170> PatentIn version 3.5

<210> 1
<211> 4133
<212> DNA
<213> Homo sapiens

<400> 1

caaaatgtgg tggtatgtgt gggcagagtg gctagtcatg aagtacttac tggcttccct 60
gattgaatac agagtgaaga catctgcagc agaatttatg agagtgcaca aagaattgat 120
tctataacct tcctgccctc aagtggcttc cacctcaagt ggaaggtaag acaggccaac 180
acaatacata atagtgagta ctgagacgct gttaaaaaaa caaaaagtag cccctctcat 240
acatgggcat ttgggtcata ggatgtgatc agtgaagaaa ggcttgattg attatcaaag 300
cacaagaaga tcacagattg gcagacggag ggtatcaatc atttcctact tggaggtggg 360
gaggtagaga acactgtgca tgcctgtgtg tgtttgagtg tgtaggtttg ctggtccagg 420
ctctggcatt gtctaatata atacaagcca catatatggt ttaaaaatgt ctagtagcca 480
tatataaaag caaaaagaaa caagagaaat tacttttaat aataaatgtt atttaacctg 540
atatatccaa aatactattt caacatgtaa atcaattaca tgttattaat gagatttttt 600
tcataccaag ccttcaaaat cctgtgtgta ttttatattt gctgcacatt taacacctac 660
agccactttt caagtgttca agagccacag gtggcttgtg gtgactgtac tggctggcac 720
agctctggag gtgccaccac cagcttctct ggagggagta tggggagaaa agacagatct 780
tggccaagag gaaccattat ctctggttaa cctcttgctc ctgccgggaa aaaaaaaatc 840
acttctgaca gatggcagct tatctctctc tcactctatt aactctctct cttaaaaaaa 900
gacaatcaac caaaatgatc actaaccaag atggggttcc tgggaagtgg cttgtagccc 960
actacctaat ttacttcttg tggttttccc tggcttggga actgccaaca ctttcacttc 1020
ttttcctcag tctttgctgg aaaggacaga agcagaccaa tgataacccc tccacggcca 1080
gggcctaatg ctgagcttgt ttctcatggt attttcttgc tccttagggc agttgttcac 1140
aaatctgaaa agacagcctc cctttttttct cctgcttctc agaaggcctc tgctgagcac 1200

gatctctgtg ggggtaggag ctcggcatgt gtgtattgac agacaggctc atgccctttc 1260
tcgtggctat tttcagctct ctggctttat gtttcacttt ctcttcttaa ttagatacta 1320
gaagaacaaa tcttgaagac caaattccct ttctcctccc gtattcagtt gggagtggaa 1380
taaaataatt tagaatttat gaggagccca gggtgaagag aaagttaatc tacacaaaaa 1440
agtcagtcca agagggggaa attctggcag aatgtccccc aggtcctttc cagcatcttt 1500
agtcggacag aacgttcctt cctttgcaga actcagctca ctctatgttc tggggtgctt 1560
ccacgttgtt tgaagaatcc acggctccag tgtcgaatgt gtaccggaca gagggacggg 1620
ttggcttctg ggacagtgga aagggaagaa gcttgggttc tctttcccca gtactaggct 1680
tcctctagct tctgtccaag tgagggagtc tggaagcaga ttcggggaga cctttttttcc 1740
tgttttactc tagagctaac aaaaaaaccc tgaagttggg tggggtcaaa aagggctcat 1800
tccacataca attaagtcca gaaatcagtc ccccaatccg ggaaaggcga tcctttagag 1860
acccaccccc caagcccccg ccccggagct cacccctcag ttccctcagc ccctagcccc 1920
tgcttcgctg aaggggccgg gcttgggggc agggcggggc cgggggcggg gcctcgccgg 1980
ctcgcggccg gtcgccttga cgaccgcagc aagatggaga cgctgccagg cttgctgcag 2040
cggcccgacc ccggggcgct tagcgcagcg cagctggagc agctgcgaaa attcaaggtg 2100
ggtgcgcccg cgcccccatc cagcgtccac caaagtgtag ctgccccagg actggggccc 2160
cagactggga agagagccgc cgagagaagg ggagcgggag cctgttccct gataaagtgg 2220
ggagggcgca ggggagaccc ccggcggacc tctaggcgtc tgatcccagc acagagcgcg 2280
acgggcatct gagagtgcag gaagaactaa ctcagggact cggccagaga aatgggccga 2340
aattactgcc agacacatcc gaccttagta aaggcccctc atcctgaaca agcggcagaa 2400
tgatatttta tctgtaagaa gatgggtggc ttaaactttc aagcatcctg ggaaaagact 2460
tgggttaaat gcgcacgccg tttcccgcag cccttcacat ctccagggct gaaccctgaa 2520
tctgtctcct aatggaagtg accctttgtt cttgcccccc agattcagac tcggattgct 2580
aacgaaaagt acctaaggac ccacaaagaa gtagagtggc tcataagtgg tttcttcagg 2640
taggtggttt tccaggctct gggatttgtg gcacgcaggg ttttcttcgt tatctctaga 2700
ggtttccttc agttatctct aaattcttaa aaatatgtct attcatttac tttaaccata 2760
aggataaata catacctttat gacacttgga agagggaatt tggcgttgcg aaacccaaca 2820
gccatccact gggtgtagaa cactattctc aaccatagga gcttaacaaa atcactagtt 2880
gaacttaaaa caaaaatcag ttgtctggtt ggcccttccc tacttgaaaa cattactgta 2940
gaaaaattcc aagggttgga agccactctt ttcttgggga agagaagaca atttaccagc 3000
gtatcctgga atatgttctt tccttccccc accctccttt cagcctggca tttgcttgaa 3060
tggaaacata tcctaatggg atgaagaagg agaattaaaa aaataaatca cggagcatca 3120

```
gtggatgaaa acaatagagc tatctcaaat gctgtttggt tatagacaaa tagacaaggt     3180
ttaaataatt ataatggcac ccatctccag gcattgtgct gttcccctac attagcactt     3240
accacactgt attgtaattg agctgtcaat cttctgttac tggtccaaag ctccgagctt     3300
gtggagtgtg tttgtgttct cagtgcccac gcagtgtgtg gcatcttgga acacttttgc     3360
tgatcctccc agcacctcca caaaagacag gtgattgtca tttttatttt ataagtgaga     3420
aaatttaagc ttagaaaggt gaagtacctt cccaaagttg ctgaaccagg attcaaaccc     3480
ctggttttcc cactgtattg tgctgtaagc agtcggtccc catttcccct ctggactgtg     3540
tgcttatagc cagaggtggg ccctcccttg tcttctcaaa agttggcgag gtcaggcaag     3600
gtggctcatg cctgtaatcc cagcactttg ggaggccgag gcgggtggat cacctgaggt     3660
cgggagttcg agaccagcct gaccaacatg gtgaaacccc gtctctacta aaaatacaaa     3720
attagccagg cgtggtggca catgcctgta atcccagcta cttgagaggc tgaggcagga     3780
gaatcgcttg aacccaggag gcaaaggttg cagtgagctg ggattgctcc attgcactct     3840
agcctgggca acaagagcaa aactccatcg caaacaaaca aaaaaaagtt ggccaggcag     3900
atgcgttggc tcacgcctgt gatcccagca ctttgggagg ctgagagggg tggatcactt     3960
gaggccagga gttcgagacc tgcctggcca acatgaggaa accctgtctc tactaaaaat     4020
acaaaaatta gtcaggcgtg gtggcacgtg cctgtaatcc cagctacctg ggagactgag     4080
gcaggagaat cgcttaaacc tgggaggcag aggttgcagt gagccgagat tgc            4133
```

<210> 2
<211> 133
<212> DNA
<213> Artificial Sequence

<220>
<223> bisulfite-treated polynucleotides

<400> 2

```
cgaucguagu aagatggaga cgutguuagg uttgutguag cgguucgauu ucggggcgut       60
tagcguagcg uagutggagu agutgcgaaa attuaaggtg ggtgcguucg cguuuuuatu      120
uagcgtuuau uaa                                                         133
```

<210> 3
<211> 23
<212> DNA
<213> Artificial Sequence

<220>
<223> primer

<400> 3

cgatcgtagt aagatggaga cgt 23

<210> 4
<211> 25
<212> DNA
<213> Artificial Sequence

<220>
<223> primer

<400> 4
ttaataaacg ctaaataaaa acgcg 25

<210> 5
<211> 33
<212> DNA
<213> Artificial Sequence

<220>
<223> primer

<400> 5
gggatattaa gtggagttat tttggtttta gtt 33

<210> 6
<211> 21
<212> DNA
<213> Artificial Sequence

<220>
<223> primer

<400> 6
ccctcccaac atccttccta a 21

<210> 7
<211> 133
<212> DNA
<213> Homo sapiens

<400> 7

```
cgaccgcagc aagatggaga cgctgccagg cttgctgcag cggcccgacc ccggggcgct    60
tagcgcagcg cagctggagc agctgcgaaa attcaaggtg ggtgcgcccg cgcccccatc   120
cagcgtccac caa                                                      133
```